Europäisches Patentamt

(19) European Patent Office

Office européen des brevets

(11) Publication number: **0 241 025**
**A1**

# (12) EUROPEAN PATENT APPLICATION

(21) Application number: **87105235.3**

(22) Date of filing: **08.04.87**

(51) Int. Cl.³: **G 01 N 1/30**

(30) Priority: **08.04.86 US 849553**

(43) Date of publication of application:
**14.10.87 Bulletin 87/42**

(84) Designated Contracting States:
**CH DE ES FR GB IT LI**

(71) Applicant: **MELOY LABORATORIES, INC.**
**6715 Electronic Drive**
**Springfield Virginia 22151(US)**

(72) Inventor: **Fennell, Robert Henry, III**
**6107 Eagle Landing Road**
**Burke Virginia(US)**

(72) Inventor: **Ponsell, Karen**
**7031 Skyles Way Apt. T4**
**Springfield Virginia(US)**

(74) Representative: **Patentanwälte Grünecker, Kinkeldey,**
**Stockmair & Partner**
**Maximilianstrasse 58**
**D-8000 München 22(DE)**

(54) Method and substrate for immunofluorescent microscopy.

(57) This invention relates to a method for the fixation of a cell substrate for immunofluorescence microscopy comprising contacting the cell substrate to be fixed with at least one solution of methanol and acetone under conditions of time and temperature sufficient to fix the cell substrate.

METHOD AND SUBSTRATE FOR IMMUNOFLUORESCENT MICROSCOPY

This invention relates to the field of immunofluorescent microscopy. More specifically, the invention provides a novel method for substrate fixaticn resulting in a significantly improved substrate for immunofluorescent microscopy.

Ever since the now classic studies of Albert Coons which demonstrated that fluorescent dyes (e.g. fluorescein and rhodamine) could be conjugated to antibodies without destroying the antigen reactivity of the antibody and that such a fluorescent antibody could be detected when reacted with antigen in a tissue sample by means of a U.V. microscope, the power of immunofluorescent assays has been appreciated. A variety of assay protocols have been developed. The so-called "Direct Test" is performed when the antibody to the tissue substrate is itself conjugated to the fluorochrome and applied directly to the tissue, said tissue having been previously attached to a microscope slide. For example, to demonstrate the distribution of a nuclear autoantigen with an autoantibody present in the serum of a patient with progressive systemic sclerosis, the immunoglobulin is isolated from the patient's serum, conjugated with fluorescein, and applied to a tissue sample on a slide. When observed through a U.V. microscope, brightly fluorescent nuceloli can be seen within the cell nucleus.

The so-called "Indirect Test" is performed when unlabelled antibody is contacted with the tissue substrate, and then visualized indirectly by reaction with a fluorochrome labelled anti-immunoglobulin.

Continuing with the example mentioned above in reference to the direct test, the indirect test can be used to detect the presence of the anti-nucleolar autoantibody in the patient's serum. Accordingly, a tissue sample is placed on a slide, then a serum sample from the patient is added and washed well. If the patient's serum contains the antibody, it will be retained on the slide by virtue of being bound by the autoantigen. This antigen-antibody complex is then indicated by reacting the complex with a second antibody which has been fluorochrome labelled. The indicator antibody could be a rabbit anti-human immunoglobulin serum. The indirect technique has numerous advantages: 1) it results in a brighter fluorescence than the direct test since several anti-immunoglobulin molecules can bind to each of patient's serum antibodies, thereby amplifying the signal; 2) since the conjugation reaction of fluorochrome to antibody is time consuming and expensive, much time and money can be saved when a large number of sera have to be screened, because it is necessary only to prepare a single labelled reagent, i.e., the anti-immunoglobulin rather than each serum antibody separately; 3) by employing conjugates of antisera to individual heavy chain isotypes, the distribution of subclasses can be evaluated; 4) complement fixation can also be tested by using fluorescent anti-complement reagent; and 5) even greater sensitivity is possible by adding a tertiary antibody. In the latter case the tissue substrate is attached to the slide the serum sample is added, followed by unlabelled rabbit anti-human immunoglobulin, the entire complex then indicated by reaction with a fluorochrome labelled goat anti-rabbit immunoglobulin.

A third assay system, the so-called "sandwich assay" is also known. In this procedure, a cell possessing

0241025

antibodies on its surface, e.g., a plasma cell which manufactures the antinuclear antibody _supra_, is affixed to a slide. The nucleolar antigen is then added and the complex is detected by a second fluorescent-labelled antinucleolar antibody. The name of the test derived from the fact that the antigen is sandwiched between the two antibodies of interest. The sensitivity of this test can be increased by the tertiary methods discussed above.

As with most immunological techniques as sensitivity is increased, specificity becomes progressively reduced. This problem even though recognized early in the practicing of immunofluorescent microscopy, continues to plague researchers to this day.

This invention provides a novel method for fixing tissue substrates on an insoluble surface such as a glass slide. The resultant fixed substrate is characterized by both optimal specificity and sensitivity.

This invention provides a method for the fixation of a cell substrate for immunofluorescence microscopy comprising contacting the cell substrate to be fixed with at least one solution of methanol and acetone under conditions of time and temperature sufficient to fix the cell substrate.

This invention also provides a methanol/acetone fixed cell substrate for immunofluorescence microscopy characterized by:

    a) a high sensitivity usually associated with acetone fixation;

    b) visually crisp patterns associated with specific antigens to which antisera is directed; and

    c) low non-specific fluorescence and high specificity usually associated with alcohol fixation.

In a further embodiment this invention provides an improved kit for the detection of ANA by immunofluorescence microscopy comprising a carrier being compartmented to receive a series of containers in close confinement which comprises:

a first container containing a methanol/acetone fixed HEp-2 cell substrate characterized by

a) a high sensitivity usually associated with acetone fixation;

b) visually crisp patterns associated with specific antigens to which antisera is directed; and

c) low non-specific fluorescence and high specificity usually associated with alcohol fixation.

a second container containing positive ANA control serum;

a third container containing negative ANA control serum;

a fourth container containing an FITC conjugated antibody and counterstrain;

a fifth container containing phosphate buffer saline (PBS) salts; and

a sixth container containing buffered glycerol.

Immunofluorescent microscopy has been employed in a variety of diagnostic and detection applications including but not limited to the localization of cell receptors, and cellular antigens, identification and quantitation of circulating antibodies in a variety of autoimmune diseases such as rheumatoid arthritis (RA) systemic lupus erythematosus (SLE) and mixed connective tissue disease (MCTD) and Primary Biliary Cirrhosis, Hashimoto's thyroiditis, Pernicious anemia, etc. as well as antibodies to infectious agents.

Although this invention has wide application to any type of immunofluorescent microscopic technique which requires optimal sensitivity and specificity, the invention will be described with reference to detection of antinuclear antibodies (ANA). The detection of these antibodies is one of the more commonly performed assays in clinical laboratories. The importance of these antibodies and their cognate antigens iin the diagnosis and prognosis of autoimmune disease has been steadily increasing.

The advent of immunofluorescent techniques allowed for the identification of a group of autoantibodies which were regularly associated with SLE. These autobodies have as their target the nuclei of the patient's own cells and became known as antinuclear antibodies (ANA). There are a wide variety of antigenic targets which exist in the nuclease. In SLE alone, autoantibodies against such nuclear components as nucleo-protein, nuclear glycoprotein, double-stranded DNA (dsDNA), single-stranded DNA (ssDNA), double-stranded RNA (dsRNA), single-stranded RNA (ssRNA), polynucleotides, and a group of antigens known as extractable nuclear antigens (ENA) have been identified. Table 1 provides an overview of the antigenic reactivity of a number of ANA's. As in evident from Table 1 the pattern of fluorescence will provide clues to the nature of the autoantibody and provides the technician with diagnostic and prognostic information.

## TABLE 1 - REACTIVITY OF VARIOUS ANAs

| Nature of Antigen | Disease Association | Pattern Observed on Indirect Immunofluorescence | |
|---|---|---|---|
| 1. Double stranded DNA (dsDNA) | SLE | Rim and/or homogeneous | 1 |
| 2. Single stranded DNA (ssDNA) | Rheumatic nonrheumatic D | Not Detected | 2 |
| 3. Both dsDNA and ssDNA | SLE others | Same as 1 | 3 |
| 4. DNA/histone complex | LE cell | Same as 1 | 4 |
| 5. Histones alone | SLE, drug induced SLE | Homogeneous and/or Rim | 5 |
| 6. Glycoprotein | SLE | Speckled | 6 |
| 7. RNA-protein complex | MCTD | Speckled | 7 |
| 8. Ribosomal fraction | SLE | Nucleolax Cytoplamsa | 8 |
| 9. Extractable Nuclear | Scleroderma | Atypical speckled | 9 |
| 10. 4-6S RNA | Progressive Systemic Sclerosis | Nucleolar | |

It is now appreciated that in order to achieve repeatable and reliable patterns the choice of substrate and its preparation is extremely important. A variety of substrates have been reported as being useful, these include: human, dog, mouse, rat, rabbit, and chicken kidney and liver, guinea pig, and Chinese baby hamster kidney,; human skin,; human leukocytes,; and tissue culture cells such as HeLa, (human cervical carcinoma), KB (human oropharyngeal carcinoma) HEp-2 (human laryngeal carcinoma), human embryonic lung fibroblasts, human B lymphoid cells, Vero (african green monkey), BHK-21, (baby hamster kidney) and mouse Ehrlich ascites and myeloma cells; and the hemoflagellate _Crithidia lucilliae_. Of the substrates listed above, the HEp-2 (human epithelial) cell line has become increasingly popular because they contain large nuclei and nucleoli making pattern interpretation substantially easier and are capable of resolving certain ANAs which are undetectable in tissue sections.

Although the fluorescent antinuclear antibody (FANA) technique is quite useful, it would be desirable to overcome several negative features of the assay. First, the high sensitivity of the technique leads to the detection of ANAs in patients with a wide variety of rheumatic and nonrheumatic diseases, in elderly individuals without apparent disease, and in at least 2% of normal, non-elderly individuals. Second, the test may be negative in rare cases of SLE which have antibodies restricted to cytoplasmic constituents. Third, the FANA test does not detect all known ANAs. This is particularly true for sera from patients with Sjogren's syndrome, polymyositis, and scleroderma, for which negative or low-titer ANAs may be observed despite high titers of antibodies detected by other techniques. Fourth, the FANA test is not very useful in following the clinical course of systemic rheumatic diseases because it measures antibodies of many specificities and therefore does not always quantitate particularly pathogenic antibodies.

Of course a single improvement cannot be expected to remedy all of the above negative features, this invention addresses the sensitivity specificity problem.

As mentioned above, the chemicals most commonly used for the fixation of cell substrates for indirect immunofluorescence assays, for example, the ANA test, have resulted in antigen substrates that provide tests results which are either too high in sensitivity (i.e., false positives and high non-specific fluorescence) or too high in specificity (i.e., false negative results). Typically, alcohols, when used as fixatives, produce antigenic substrates with low sensitivity and high specificity. Alternatively, acetone produces high sensitivity and low specificity with high background fluorescent staining. The problem, therefore, is to produce a fixed antigenic substrate which maximizes both sensitivity and specificity, thereby providing the best overall accuracy of the assay system. The more commonly used alcohol fixatives are ethanol and methanol.

It has been surprisingly discovered that a combination of methanol and acetone provides a fixative which results in improved accuracy and clarity of reaction.

The efficacy of this combination was completely unexpected in view of the finding that the other combination of fixatives, e.g., ethanol and acetone, acetic acid and methanol, acetic acid and ethanol, acetone and acetic acid either combined or in sequential fixative steps were without benefit in improving sensitivity and clarity.

This invention provides a fixation process which optimizes both specificity and sensitivity of the indirect immunofluorescence test. It was found that by combining two fixatives - acetone and the alcohol, methanol - the above purpose can be achieved. Furthermore, the sensitivity and specificity of the immunofluorescence assay can be modulated by varying the ratio of methanol to acetone in the fixation process. As exemplified herein, it was found that for one human epithelial cell line, HEp-2, when fixed with a mixture of methanol/acetone in a certain ratio, provides an ANA test that has low nonspecific fluorescence with normal human serum, yet demonstrates the clarity and sensitivity of positive reactions commonly found with HEp-2 cells fixed in only acetone.

The advantage of the unique combination of acetone and methanol in a certain ratio is that an antigenic substrate can be produced which offers: (1) the high sensitivity of the assay usually associated with acetone fixation, (2) visually crisp patterns associated with the specific antigen to which the antisera are directed, and (3) low non-specific fluorescence and high specificity provided by alcohol fixatives.

It has been discovered that a combination of methanol and acetone in a ratio of from about 1:3 to about 10:1 is useful. The specific ratio for a particular use is easily determined as taught in the Example, keeping in mind that sensitivity generally increases with increasing acetone concentration and generally decreases with decreasing acetone concentration.

While minor amounts of water can be present in the fixative of this invention, the acetone and methanol employed are preferably essentially dry, i.e., containing less than about 1% of water by volume.  The fixing method is useful with a variety of substrates including but not necessarily limited to the substrates discussed above.  Substrate as used herein refers to not only tissue substrates as for example from tissue sections, but also cell suspension as obtained, for example, from tissue cultures and can also apply to cells specifically treated with chemicals to ehance the formation of specific antigens or infected with bacteria or viruses to detect antibodies to viral or bacterial antigens.

The fixation can be run at a temperature from about -70°C to up to about 20°C.  It is preferred to carry out the fixation at a temperature of about 0°C to about 5°C.

The time of fixation can run from about 2 to about 20 minutes, with about 5 minutes being preferred.

When a plurality of samples are being run, it is preferred that the slides having the substrate afixed thereto be dipped into first container containing a methanol acetone solution for about 20 seconds at a temperature of about 2-4°C prior to the fixation step.  This procedure prevents the accumulation of salts in the fixation solution.

The invention is further described by means of the following non-limiting Example.

## EXAMPLE 1

This Example illustrates the beneficial result obtained with a methanol-acetone fixation procedure when ANA are detected by immunofluorescence microscopy.

The HEp-2 cell line (ATCC CCL 23) was cultured as a monolayer on Basal Medium (Eagles with Hanks salts) in 10% fetal calf serum. The cells were harvested and applied to the surface of a microscope slide. The cells were allowed to attach to surface of the slides by incubation at 37°C in a humidified incubator. The slides were then washed by dipping 2-3 times in 0.85% saline at room temperature, dipped into the fixative for 20 seconds and fixed in a fixative under various conditions as given in Table II. The slides were allowed to dry at room temperature for about 10 minutes. Then the slide was dried in a vaccum dryer overnight, packaged in a foil pouch with dessicant and stored at -20°C until ready for use.

A fluorescent ANA test was conducted according to the following protocol:

The reagents were equilibrated to room temperature serum samples were diluted 1:40 in phosphate buffered saline (PES) and applied to the substrate. Samples were incubated in a moist chamber for 30 minutes at room temperature, each well rinsed and washed 2x5 minutes in PBS. FITC-conjugated anti-human globulin/counterstrain was applied. Samples were incubated in a moist chamber for 30 minutes at room temperature, each well rinsed and washed 2x5 minutes in PBS. A mounting medium and coverslip were applied and examined at 490 nm excitation and 530 nm emission fluorescence microscope.

The results from various fixing protocols are given in Table II. As can be seen the methanol acetone fixatives yield superior results.

|  | SAMPLE | | | | | | | | CONTROLS | |
|---|---|---|---|---|---|---|---|---|---|---|
|  |  |  |  |  |  |  |  |  | I | J |
| **Methanol** 4° 5 min | 3 | 2-3$^+$ | 2$^+$ | 2-3$^+$ | 1$^+$ | $^+/_-$ | 1$^+$ | 1$^+$ | N | N$^+/_-$ |
| **Ethanol** 4°C 5 min | $^+/_-$ | $^+/_-$ | N | 2$^+$ | $^+/_-$ | N$^+/_-$ | $^+/_-$ | N | N | N |
| **Acetone** 4°C 10 min | 3$^+$ | 3-4$^+$ | 2-3$^+$ | 3$^+$ | 2$^+$ | 1$^+$ | $^+/_-$ | $^+/_-$ | $^+/_-$ | $^+/_-$ |
| **MeOH/AC 2:1** 4°C 5 min | 1-2 | 1$^+$ | $^+/_-$ | 1-2$^+$ | 1$^+$ | $^+/_-$ | N$^+/_-$ | N$^+/_-$ | N | N |
| **MeOH/Ac 1:2** 4°C 5 min | 3-4$^+$ | 3-4$^+$ | 3$^+$ | 2-3$^+$ | 2-3$^+$ | 1$^+$ | 1$^+$ | 1$^+$ | N$^+/_-$ | N$^+/_-$ |

strong pos. homo = strong positive homogeneous pattern

pos. nuc. = positive nucleolar pattern

wk pos. homo. = weak positive homogeneous pattern

wk pos. S.A. = weak positive spindle apparatus

wk pos. cent. = weak positive centromere pattern

NHS = normal human serum

N = no fluorescent

N$^+/_-$ = slight fluorescent no pattern discernible

$^+/_-$ = slight fluorescent no pattern discernible

1$^+$, 1-2$^+$, 2$^+$, 2-3$^+$, 3$^+$, 3-4$^+$ increasing fluorescence

What is Claimed is:

1. A method for the fixation of a cell substrate for immunofluorescence microscopy comprising contacting the cell substrate to be fixed with at least one solution of methanol and acetone under conditions of time and temperature sufficient to fix the cell substrate.

2. The method according to Claim 1 wherein the cell substrate is attached to a solid support prior to fixation.

3. The method according to Claim 2 wherein the solid support is a microscope slide.

4. The method according to any of the Claims 1 - 3 wherein the cell substrate is selected from the group consisting of a cell suspension, tissue section, chemically treated cells and infected cells or bacteria.

5. The method according to any of the Claims 1 - 4 wherein the ratio of methanol to acetone is from about 1:3 to about 10:1.

6. The method according to any of the Claims 1 - 5 wherein the ratio is about 1:2.

7. The method according to any of the Claims 1 - 6 wherein the cell substrate is contacted with the methanol/acetone solution for about 1 to 20 minutes at a temperature of from about -70°C to about 20°C.

8. The method according to any of the Claims 1 - 7 wherein the time is about 5 minutes and the temperature is about 0-5°C.

9. The method according to any of the Claims 1 - 8 including the further step of drying the fixed cell substrate.

10. The method according to any of the Claims 1 - 9 wherein the contacting step comprises dipping the cell substrate into methanol: acetone (1:2) for 20 seconds at 0-5°C followed by fixing the substrate in methanol : acetone (1:2) at 0-5°C for 5 minutes.

11. A methanol/acetone fixed cell substrate for immunofluorescence microscopy characterized by:

    a) a high sensitivity usually associated with acetone fixation;

    b) visually crisp patterns associated with specific antigens to which antisera is directed; and

    c) low non-specific fluorescence and high specificity usually associated with alcohol fixation.

12. The substrate when prepared by the process of any of the Claims 1 - 10.

13. A kit for the detection of antinuclear antibodies comprising a carrier being compartmented to receive a series of containers in close confinement which comprises:

    a first container containing a methanol/acetone fixed HEp-2 cell substrate characterized by

    a) a high sensitivity usually associated with acetone fixation;

    b) visually crisp patterns associated with specific antigens to which antisera is directed; and

    c) low non-specific fluorescence and high specificity usually associated with alcohol fixation.

    a second container containing positive ANA control serum;

    a third container containing negative ANA control serum;

    a fourth container containing an FITC conjugated antibody and counterstrain;

    a fifth container containing phosphate buffer saline (PBS) salts; and

    a sixth container containing buffered glycerol.

**European Patent Office**

# EUROPEAN SEARCH REPORT

Application number

0241025

EP  87 10 5235

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| A | US-A-3 546 334 (LERNER et al.) * Column 1, line 39 - column 2, line 31; column 2, lines 49-58; claims 1,3,10 * | 1-5,9 | G 01 N  1/30 |
| | --- | | |
| A | EP-A-0 094 603 (AMERICAN HOECHST CORP.) * Page 6, line 14 - page 7, line 9; page 7, lines 24-30; page 8, lines 23-29; page 10, lines 4-22; page 12, line 18 - page 13, line 3; page 13, lines 7-26; page 14, line 16 - page 15, line 9; page 16, line 23 - page 17, line 29; claims 1,6,7,9 * | 1,4,9, 13 | |
| | --- | | |
| A | WO-A-8 300 877 (ICL SCIENTIFIC) * Page 4, lines 20-26; page 5, lines 12-24; page 6, lines 24-34; page 7, lines 27-34; claims 1,8-12,16 * | 3,4,7, 9 | |
| | | | **TECHNICAL FIELDS SEARCHED (Int. Cl.4)** |
| | | | G 01 N  1/00 G 01 N  33/00 |
| | --- _ -/- | | |

The present search report has been drawn up for all claims

| Place of search | Date of completion of the search | Examiner |
|---|---|---|
| THE HAGUE | 09-07-1987 | FORMBY N.M. |

## DOCUMENTS CONSIDERED TO BE RELEVANT

Page 2

| Category | Citation of document with indication, where appropriate, of relevant passages | Relevant to claim | CLASSIFICATION OF THE APPLICATION (Int. Cl.4) |
|---|---|---|---|
| A | JOURNAL OF IMMUNOLOGICAL METHODS, vol. 88, no. 1, 3rd April 1986, pages 115-120, Elsevier Science Publishers B.V., Amsterdam, NL; H. SCHMITZ et al.: "Specific enzyme immunoassay for the detection of antibody to HTLV-III using rheumatoid factor-coated plates" * Page 117, column 1, line 40 - column 2, line 8 * | 1-4 | |
| A | EP-A-0 159 603 (J. KONRATH et al.) * Page 2, lines 7-18; page 3, line 25 - page 4, line 7; page 6, lines 1-26; page 7, lines 15-19; claims 1-4,7 * | 1-4,9 | |

TECHNICAL FIELDS SEARCHED (Int. Cl.4)

The present search report has been drawn up for all claims

| Place of search | Date of completion of the search | Examiner |
|---|---|---|
| THE HAGUE | 09-07-1987 | FORMBY N.M. |